# EUROPEAN PATENT SPECIFICATION

(11) **EP 4 401 639 B1**
(45) Date of publication and mention of the grant of the patent: **04.03.2026**
(21) Application number: 21777330.8
(22) Date of filing: 16.09.2021
(51) Int. Cl.: A61B 8/08, A61B 8/00

(54) **SEGMENTED ULTRASOUND PROBE**
SEGMENTIERTE ULTRASCHALLSONDE
SONDE ULTRASONORE SEGMENTÉE

(43) Date of publication of application: 24.07.2024
(73) Proprietor: Brainlab SE, 81829 München (DE)
(72) Inventor: STEINLE, Wolfgang, 81829 Munich (DE); HABERHAUSEN, Jannis, 81829 Munich (JP); FRIELINGHAUS, Nils, 81829 Munich (JP)
(74) Representative: SSM Sandmair
(86) International application number: PCT/EP2021/075483
(87) International publication number: WO 2023/041162

(56) References cited:
- EP-B1- 2 726 902
- WO-A1-2018/054969
- JP-A- 2002 033 708
- JP-A- 2003 075 218
- US-A1- 2010 268 503
- US-A1- 2015 157 294
- US-A1- 2019 328 359
- US-A1- 2021 186 462

## Description

### FIELD OF THE INVENTION

The present invention relates to an ultrasound probe having a segmented construction for being assembled individually to obtain a desired probe geometry for meeting requirements of a large variety of different applications for an ultrasound probe, a corresponding computer-implemented method for determining emission characteristics and/or reception characteristics of such ultrasound probe and a corresponding computer program.

### TECHNICAL BACKGROUND

Ultrasound imaging is an imaging modality that uses high-frequency sound waves to generate images of anatomical structures inside a patient's body. Usually, a hand-held probe comprising an ultrasound transducer for converting electrical energy into sound energy and vice versa is brought into direct contact with the skin or is placed inside a body opening, such that sound waves generated by the transducer are transmitted into the body. Based on sound waves reflected from structures within the body, which are received by the transducer again, an ultrasound image is generated.

As diagnostic or interventional procedures often require a specific geometry of an ultrasound probe, numerous ultrasound probes of a different geometry need to be kept available for different applications. In some applications, only a narrow window is available for the ultrasound probe, for example between the ribs or in a small opening in the shell (craniotomy). In other applications it is desirable to cover a large area or volume which for example includes vascular structures. EP2726902B1 relates to a transducer assembly for an echoscope, comprising a first row of ultrasonic transducers and a second row of ultrasonic transducers in the form of a modular unit.

The present invention provides an ultrasound probe that can be assembled in different configurations and geometries so as to suit a vast number of different ultrasound applications. Moreover, the present invention allows to use a concept called "multiple aperture ultrasound" in which an object is imaged via a plurality of transducers being disposed separately at multiple locations on the body, wherein one or more transducers receive soundwaves generated by one or more other transducers.

The present invention can be used for any kind of 2D or 3D ultrasound examination procedures e.g. in connection with medical tracking and navigations systems such as Kick^{®} or Curve^{®}, both products of Brainlab AG.

Aspects of the present invention, examples and exemplary steps and their embodiments are disclosed in the following. Different exemplary features of the invention can be combined in accordance with the invention wherever technically expedient and feasible.

### EXEMPLARY SHORT DESCRIPTION OF THE INVENTION

The invention is defined by the appended claims. In the following, a short description of the specific features of the present invention is given which shall not be understood to limit the invention only to the features or a combination of the features described in this section.

In general, the ultrasound probe according to the present invention features a segmented setup, wherein a plurality of individual segments can be assembled for form an ultrasound probe of a desired geometry and form factor. Moreover, the segments may be used individually without being rigidly connected to each other, but still forming part of an ultrasound probe or device as a technical entity.

### GENERAL DESCRIPTION OF THE DISCLOSURE

In this section, a description of the general features of the present disclosure is given for example by referring to possible embodiments of the invention.

In general, the disclosure reaches the aforementioned object by providing, in a first aspect, an ultrasound probe for emitting and/or receiving ultrasound waves, comprising at least one probe segment that includes:
- a housing,
- at least one transducer element which is at least partially received in the housing and is adapted to convert electrical energy in sound energy and/or to convert sound energy in electrical energy;
- at least one signal interface connected to the at least one transducer element and adapted to transmit signals corresponding to the emitted and/or received ultrasound waves;
- at least one mounting interface on the housing, which is adapted to transmit a mechanical load to and from the housing, and in particular is adapted to connect to a corresponding mounting interface of a similar probe segment.

In other words, the ultrasound probe comprises at least one probe segment, wherein any number of further probe segments can be added to form an ultrasound probe of a desired size and geometry. For this purpose, the probe segments feature mounting interfaces that connect with each other and allow for a rigid but still releasable connection of multiple probe segments.

For example, the probe segments may be coupled to each other via any sort of positive fit and/or force fit provided by the corresponding mounting interfaces. For example, a mounting interface of one probe segment is configured as dovetail-recess, whereas a mounting interface of another probe segment is configured as a corresponding dovetail-protrusion adapted to be inserted into the dovetail-recess, such that the corresponding probe segments can be released from each other again only by moving the dovetail-protrusion out of the dovetail-recess. Moreover, corresponding mounting interfaces may provide a magnetic force to hold corresponding probe segments together. It is also conceivable that the mounting interfaces provide some sort of snap-in locking mechanism to bind probe segments together.

Each one of the probe segments further includes at least one signal interface which allows for transmitting signals as to the emitted and/or received ultrasound waves between individual probe segments. The signal interface may also transmit signals between the probe segments and a signal unit for controlling the transducer elements of the probe segments to emit ultrasound waves in a desired manner, and for evaluating ultrasound waves received by transducer elements of the probe segments. While it is generally conceivable that such signal interface may be implemented as a wireless interface providing a radio communication between corresponding elements of the ultrasound probe or an external device, the signal interface may also comprise at least one electrical contact between those elements. Moreover, the signal interface may be integrated in the mounting interface or may from a part thereof.

Each one of the probe segments may comprise any number of transducer elements arranged in any desired spatial configuration. For example, at least one probe segment may include a row of transducer elements for generating and receiving ultrasound waves, that may be disposed in a linear arrangement. In general, such linear or one-dimensional arrangement of transducer elements allows for a two-dimensional ultrasound image being generated from ultrasound waves received by the individual transducer elements of the one-dimensional transducer array. One or more of the probe segments may include more than one linear arrangement of transducer elements, or form such arrangement when being attached to each other, wherein at least one linear arrangement is adapted or controlled to generate ultrasound waves, and at least one further linear arrangement is adapted or controlled to receive ultrasound waves. In case those arrangements are oriented at a predefined angle with respect to each other, for example at 90°, the respective probe segments allow for generating 3D-ultrasound images. One or more of the probe segments may also feature a matrix or two-dimensional arrangement of transducer elements, which allows for a three-dimensional ultrasound image being generated from ultrasound waves received by the individual transducer elements of the two-dimensional array. The transducer elements of such two-dimensional array or matrix may be controlled individually or via an RCA (row-column-addressing)-scheme as described further below.

Further, the transducer surface, i.e. the surface defined by the one or more transducer elements which is brought into contact with the patient's anatomy can be one of a planar surface, a one-dimensionally curved surface or a two-dimensionally curved surface. The radius of curvature can be appropriately selected depending on the desired use of the specific probe segment.

Any number of the transducer elements of one or more segments can be configured or controlled to either convert electrical energy in sound energy, i.e. generate ultrasound waves in depending on a received electrical signal, or being configured or controlled to convert sound energy in electrical energy, i.e. to convert ultrasound waves received from the patient's body into an electrical signal that is later processed to generate an ultrasound image.

It is conceivable that some transducer elements of a probe segment are adapted to generate ultrasound waves, while other transducer elements of the probe segment are adapted to receive ultrasound waves. The generating elements and the receiving elements may be arranged in any desired manner or pattern. Each one of or some of the elements may be controlled to either receive or generate ultrasound waves, or both. It is thus possible to create ultrasound waves having a desired wavefront by controlling specific transducer elements of one or more probe segments, wherein the ultrasound waves reflected back to the ultrasound probe are received by the same and/or other transducer elements of these probe segments. In a specific example, one or more probe segments of an ultrasound probe are adapted to exclusively generate ultrasound waves, wherein other probe segments of this ultrasound probe are to receive the ultrasound waves reflected within the patient's body.

In order to facilitate handling of the ultrasound probe with one or more probe segments, the housing of at least one probe segment may comprise a handle portion to be grasped by a partitioner so as to hold or move the one or more probe segments with respect to the patient's body.

Further, the signals received and sent by the transducer elements and transmitted via the signal interface can be analogue and/or digital signals. Thus, the at least one signal interface of the at least one probe segment may
- transmit signals to the probe segment, which excite at least one predefined transducer element to emit ultrasound waves; and/or
- transmit signals from the probe segment, which describe ultrasound waves received by at least one predefined transducer element.

As was mentioned before, the ultrasound probe comprises at least one probe segment, but may be enlarged as desired by adding any number of additional probe segments, which can directly connect to each other, but may also be handled independently from each other while still belonging to the same ultrasound probe as a physical entity. Thus, the present disclosure also relates to an ultrasound probe assembly that may comprise a plurality of selectively and mechanically interconnectable probe segments as described above, wherein the probe segments may be selectively interconnectable via the mounting interfaces. The mounting interfaces of the respective probe segments may further interconnect with each other such that the interconnected probe segments define a continuous probe surface without gaps or steps between the transducer elements.

In particular, the ultrasound probe may comprise only one probe segment. In another example, the probe may comprise any number of probe segments, particularly 2 to 4 probe segments, which are detached from each other so as to form a multiple aperture ultrasound system as described before. In another example, the ultrasound probe may comprise any desired number of probe segments rigidly coupled to each other, wherein two or more probe segments directly connect to each other, and/or wherein two or more probe segments are rigidly connected via a spacer element as described further below.

In a further example, the ultrasound probe may comprise a plurality of probe segments which are coupled to each other in a non-rigid manner, such that these probe segments can still be adjusted with respect to each other. For example, the coupling between two respective probe segments may allow for one, two or even three rotational degrees of freedom and/or for one, two or even three translational degrees of freedom between the respective probe segments. This could be achieved via at least one intermediate segment connecting between at least two respective probe segments. Further, it is conceivable that the coupling between the probe segments is elastically deformable or plastically deformable. In a specific example, a linear array of several probe segments is adapted to conform to a patient's anatomy, for example to the curved shape of a spinal column by being plastically deformed such that each one of the probe segments contacts the patient's skin with each probe segment being assigned to a specific vertebra. This may be achieved by a plastically deformable intermediate structure that connects to each one of the probe segments. While such intermediate structure may include one or more segments of a plastically deformable material, the intermediate structure may also include any number of joints or hinges which allow for such adjustment or "deformation".

While the signal interfaces of the probe segments may transmit signals between an external unit or device and the respective probe segments, these signal interfaces may also be used to transmit signals between the probe segments. Thus, an external unit or device may connect to only one probe segment via a signal interface thereof, while other probe segments connect to the external unit or device via the first probe segment and the signal interface thereof.

For generating, processing and/or analyzing the signals transmitted to and received from the transducer elements, the ultrasound probe may connect, via a wireless or cable data connection, to an external signal unit, but may also comprise such signal unit, for example within the housing of a first or "main" probe segment or within the handle portion. The signal unit may transmit signals to an external device or unit which for example displays ultrasound images generated from the ultrasound waves received via the probe. On the other hand, the signal unit may connect to the transducer elements of the at least one probe segment for controlling the generation of ultrasound waves as well as for processing or transmitting the signals received therefrom. In particular, such signal unit may be configured to selectively control predefined transducer elements of at least one of the plurality of probe segments to convert electrical energy and sound energy and/or to convert sound energy in electrical energy. It is generally conceivable that the signal unit is capable of addressing, i.e. controlling each transducer element individually in a "fully addressed" configuration. An easier and therefore more cost efficient addressing scheme for controlling a two-dimensional array of transducer elements is called RCA ("row column addressing scheme"). With RCA, the transducer elements are not individually controlled. Rather, full rows and/or full columns within the two-dimensional transducer element array or matrix are addressed to send and/or receive ultrasound waves. RCA allows for controlling a plurality of transducer elements to form linear senders and/or receivers within rows and/or columns of a two-dimensional array or matrix of transducer elements. Thus, a two-dimensional array or matrix of transducer elements of at least one probe segment may provide one or more linear senders and/or receivers which may further be oriented in a predefined angle, particularly parallel or perpendicularly with respect to each other.

Further, hybrid transducers may be used in which one transducer material is used for generating ultrasound waves, whereas another material is used for receiving ultrasound waves. Further, the imaging properties of a two-dimensional transducer element array may not be symmetrical such that rotating such transducer element by for example 90° results in a different ultrasound image. For a multiple probe segment probe this allows for multiple possible arrangements in which some probe segments are rotated by for example 90° with respect to the remaining probe segments, for example in a checkerboard pattern.

As already remarked above, the ultrasound probe assembly may for example comprise an intermediate segment that connects between two or more of the probe segments and holds these segments spaced apart from each other. This intermediate segments may be used to set up a specific arrangement of probe segments. For example, the probe segments may be held at a specific, predefined angle with respect to each other so as to aim at a specific location with respect to the ultrasound probe, which may be beneficial for specific applications. Further, this allows for a pre-registration of the ultrasound probe as the spatial arrangement of the probe segments is pre-defined by the intermediate segment. Thus, the spatial relationship between the sending transducer elements, the receiving transducer elements and the tissue reflecting the ultrasound waves can be easily calculated based on the predefined spatial arrangement. Further, the intermediate segment may not only connect to the mounting interfaces of the respective probe segments, but may also connect to the signal interfaces thereof so as to transmit signals between the probe segments.

In a further example, the ultrasound probe assembly may also comprise a separate handle segment which may connect to one single probe segment, for example via the mounting interface and/or the signal interface thereof, but which may also connect to two or more of the probe segments as it was described in connection with the intermediate segment.

In a specific example, one first of the plurality of probe segments may serve as a main or master segment which in particular comprises at least one of the handle portion, the signal unit and the signal interface, and at least one second of the plurality of probe segments serves as an auxiliary segment which can be selectively connected to the main segment and may further be controlled via the main segment.

At least one of the plurality of probe segments, particularly at least one of the auxiliary segments includes at least one identification feature which enables the signal unit to determine, particularly via the at least one signal interface, at least one property of emission characteristics and/or reception characteristics provided by the respective probe segment connected to the ultrasound probe assembly, particularly with respect to a point of reference assigned to the ultrasound probe assembly or a tracking marker of the ultrasound probe assembly. This allows for a pre-registration of the overall ultrasound probe, such that the emission and reception characteristics of the individual probe segments do not need to be empirically determined or calibrated, which saves time and effort when creating an individual ultrasound probe.

It becomes apparent from the description above that a basic concept of the present invention is to provide a kit of multiple probe segments which may also have different sizes or shapes, and corresponding elements such as intermediate segments or handle segments. A practitioner may individually put these elements together so as to form an ultrasound probe of a desired form factor and geometry that best suits a specific application the practitioner intends to use the ultrasound probe for.

In general, the invention reaches the aforementioned object by providing, in a second aspect, a computer-implemented medical method of determining emission and/or reception characteristics of an ultrasound probe as described herein The method comprises executing, on at least one processor of at least one computer (for example at least one computer being part of a navigation system), the following exemplary steps which are executed by the at least one processor.

In a (for example first) exemplary step, transducer emission data is acquired which describes at least one transducer element adapted to or controlled to convert electrical energy in sound energy, particularly at least one of its characteristics in converting electrical energy in sound energy and its spatial position, specifically its spatial position relative to other transducer elements of the ultrasound probe assembly. In other words, information is acquired as to the characteristics (such as form of the wave front, amplitude, frequency, spatial position including the spatial location and spatial orientation with respect to the ultrasound probe assembly) of the ultrasound waves generated by the ultrasound probe assembly when specific transducer elements convert a specific amount of electrical energy into sound energy.

In a (for example second) exemplary step, transducer reception data is acquired which describes at least one transducer element adapted to or controlled to convert sound energy in electrical energy, particularly at least one of its characteristics in converting sound energy in electrical energy and its spatial position, specifically its spatial position relative to other transducer elements of the ultrasound probe assembly. In other words, information is acquired as to how soundwaves received by transducer elements of the ultrasound probe assembly is converted into electrical energy and how the signal generated therefrom looks like.

In a (for example third) exemplary step, emission signal data is acquired which describes at least one signal transmitted to the at least one transducer element adapted to or controlled to convert electrical energy in sound energy. As it is known from the transducer emission data what sound waves look like when being generated by the transducer elements from a specific electrical signal, the emission signal data allows for determining or calculating the emission characteristics of a current setup of the ultrasound probe assembly.

In a (for example fourth) exemplary step, reception signal data is acquired which describes at least one signal received from the at least one transducer element adapted or controlled to convert sound energy in electrical energy. As it is also known from the transducer reception data, into what specific signals specific ultrasound waves are converted by the current setup of the ultrasound probe assembly, the reception signal data allows to determine or calculate from the reception signal data the characteristics of the ultrasound waves reflected within the patient's body and received by the transducer elements.

In a (for example fifth) exemplary step, correlation data is determined based on the transducer emission data, the transducer reception data, the emission reception data and the emission signal data, which describes a correlation between the emission characteristics and the reception characteristics provided by the ultrasound probe assembly. Knowledge of this correlation enables a practitioner to individually modify the setup of the ultrasound probe assembly, for example by adding individual probe segments, removing individual probe segments and/or rearranging the probe segments with respect to each other, including the relative location and/or the relative orientation.

In an example of the method according to the second aspect, the above described approach may be used for supporting assembly of an ultrasound probe assembly wherein the following steps are performed:
Properties data is acquired which describes at least one desired property of emission characteristics and/or reception characteristics provided by the ultrasound probe assembly as herein described. For example, a practitioner may define the size or shape of a desired "window" for generating an ultrasound image (such as a window between the ribs or within a craniotomy burr hole).

In a second step, assembly data is determined based on the properties data and the correlation data, wherein the assembly data describes steps for assembling an ultrasound probe assembly from the at least one probe segment, which provides the desired property of emission characteristics and/or reception characteristics. Based on the above described data, one or more setups of the ultrasound probe assembly can be calculated, which meet the requirements defined by the properties data, for example size and geometry of a desired imaging "window". Thus, instructions can be output to the practitioner as to how many and what sort of probe segments need to be put together or arranged with respect to each other in what configuration so as to acquire the desired result.

In a third aspect, the invention is directed to a computer program comprising instructions which, when the program is executed by at least one computer, causes the at least one computer to carry out method according to the first aspect. The invention may alternatively or additionally relate to a (physical, for example electrical, for example technically generated) signal wave, for example a digital signal wave, such as an electromagnetic carrier wave carrying information which represents the program, for example the aforementioned program, which for example comprises code means which are adapted to perform any or all of the steps of the method according to the first aspect. The signal wave is in one example a data carrier signal carrying the aforementioned computer program. A computer program stored on a disc is a data file, and when the file is read out and transmitted it becomes a data stream for example in the form of a (physical, for example electrical, for example technically generated) signal. The signal can be implemented as the signal wave, for example as the electromagnetic carrier wave which is described herein. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The invention according to the second aspect therefore may alternatively or additionally relate to a data stream representative of the aforementioned program, i.e. comprising the program.

In a fourth aspect, the invention is directed to a computer-readable storage medium on which the program according to the second aspect is stored. The program storage medium is for example non-transitory.

In a fifth aspect, the invention is directed to at least one computer (for example, a computer), comprising at least one processor (for example, a processor), wherein the program according to the second aspect is executed by the processor, or wherein the at least one computer comprises the computer-readable storage medium according to the third aspect.

For example, the computer-implemented method according to the second aspect does not involve or in particular comprise or encompass an invasive step which would represent a substantial physical interference with the body requiring professional medical expertise to be carried out and entailing a substantial health risk even when carried out with the required professional care and expertise.

### DEFINITIONS

In this section, definitions for specific terminology used in this disclosure are offered which also form part of the present disclosure.

The method in accordance with the invention is for example a computer implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term computer includes a server resource. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services^{™}. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz^{®}, a product of Brainlab AG. The monitor may also be the monitor of a portable, for example handheld, device such as a smart phone or personal digital assistant or digital media player.

The invention also relates to a computer program comprising instructions which, when on the program is executed by a computer, cause the computer to carry out the method or methods, for example, the steps of the method or methods, described herein and/or to a computer-readable storage medium (for example, a non-transitory computer-readable storage medium) on which the program is stored and/or to a computer comprising said program storage medium and/or to a (physical, for example electrical, for example technically generated) signal wave, for example a digital signal wave, such as an electromagnetic carrier wave carrying information which represents the program, for example the aforementioned program, which for example comprises code means which are adapted to perform any or all of the method steps described herein. The signal wave is in one example a data carrier signal carrying the aforementioned computer program. The invention also relates to a computer comprising at least one processor and/or the aforementioned computer-readable storage medium and for example a memory, wherein the program is executed by the processor.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer, a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. A step of "determining" as described herein for example comprises or consists of issuing a command to perform the determination described herein. For example, the step comprises or consists of issuing a command to cause a computer, for example a remote computer, for example a remote server, for example in the cloud, to perform the determination. Alternatively or additionally, a step of "determination" as described herein for example comprises or consists of receiving the data resulting from the determination described herein, for example receiving the resulting data from the remote computer, for example from that remote computer which has been caused to perform the determination. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

The n-dimensional image of a body is registered when the spatial location of each point of an actual object within a space, for example a body part in an operating theatre, is assigned an image data point of an image (CT, MR, ultrasound etc.) stored in a navigation system.

Image registration is the process of transforming different sets of data into one coordinate system. The data can be multiple photographs and/or data from different sensors, different times or different viewpoints. It is used in computer vision, medical imaging and in compiling and analysing images and data from satellites. Registration is necessary in order to be able to compare or integrate the data obtained from these different measurements.

It is the function of a marker to be detected by a marker detection device (for example, a camera or an ultrasound probe or analytical devices such as CT or MRI devices) in such a way that its spatial position (i.e. its spatial location and/or alignment) can be ascertained. The detection device is for example part of a navigation system. The markers can be active markers. An active marker can for example emit electromagnetic radiation and/or waves which can be in the infrared, visible and/or ultraviolet spectral range. A marker can also however be passive, i.e. can for example reflect electromagnetic radiation in the infrared, visible and/or ultraviolet spectral range or can block x-ray radiation. To this end, the marker can be provided with a surface which has corresponding reflective properties or can be made of metal in order to block the x-ray radiation. It is also possible for a marker to reflect and/or emit electromagnetic radiation and/or waves in the radio frequency range or at ultrasound wavelengths. A marker preferably has a spherical and/or spheroid shape and can therefore be referred to as a marker sphere; markers can however also exhibit a cornered, for example cubic, shape.

A marker device can for example be a reference star or a pointer or a single marker or a plurality of (individual) markers which are then preferably in a predetermined spatial relationship. A marker device comprises one, two, three or more markers, wherein two or more such markers are in a predetermined spatial relationship. This predetermined spatial relationship is for example known to a navigation system and is for example stored in a computer of the navigation system.

In another embodiment, a marker device comprises an optical pattern, for example on a two-dimensional surface. The optical pattern might comprise a plurality of geometric shapes like circles, rectangles and/or triangles. The optical pattern can be identified in an image captured by a camera, and the position of the marker device relative to the camera can be determined from the size of the pattern in the image, the orientation of the pattern in the image and the distortion of the pattern in the image. This allows determining the relative position in up to three rotational dimensions and up to three translational dimensions from a single two-dimensional image.

The position of a marker device can be ascertained, for example by a medical navigation system. If the marker device is attached to an object, such as a bone or a medical instrument, the position of the object can be determined from the position of the marker device and the relative position between the marker device and the object. Determining this relative position is also referred to as registering the marker device and the object. The marker device or the object can be tracked, which means that the position of the marker device or the object is ascertained twice or more over time.

A marker holder is understood to mean an attaching device for an individual marker which serves to attach the marker to an instrument, a part of the body and/or a holding element of a reference star, wherein it can be attached such that it is stationary and advantageously such that it can be detached. A marker holder can for example be rod-shaped and/or cylindrical. A fastening device (such as for instance a latching mechanism) for the marker device can be provided at the end of the marker holder facing the marker and assists in placing the marker device on the marker holder in a force fit and/or positive fit.

A pointer is a rod which comprises one or more - advantageously, two - markers fastened to it and which can be used to measure off individual co-ordinates, for example spatial co-ordinates (i.e. three-dimensional co-ordinates), on a part of the body, wherein a user guides the pointer (for example, a part of the pointer which has a defined and advantageously fixed position with respect to the at least one marker attached to the pointer) to the position corresponding to the co-ordinates, such that the position of the pointer can be determined by using a surgical navigation system to detect the marker on the pointer. The relative location between the markers of the pointer and the part of the pointer used to measure off co-ordinates (for example, the tip of the pointer) is for example known. The surgical navigation system then enables the location (of the three-dimensional co-ordinates) to be assigned to a predetermined body structure, wherein the assignment can be made automatically or by user intervention.

A "reference star" refers to a device with a number of markers, advantageously three markers, attached to it, wherein the markers are (for example detachably) attached to the reference star such that they are stationary, thus providing a known (and advantageously fixed) position of the markers relative to each other. The position of the markers relative to each other can be individually different for each reference star used within the framework of a surgical navigation method, in order to enable a surgical navigation system to identify the corresponding reference star on the basis of the position of its markers relative to each other. It is therefore also then possible for the objects (for example, instruments and/or parts of a body) to which the reference star is attached to be identified and/or differentiated accordingly. In a surgical navigation method, the reference star serves to attach a plurality of markers to an object (for example, a bone or a medical instrument) in order to be able to detect the position of the object (i.e. its spatial location and/or alignment). Such a reference star for example features a way of being attached to the object (for example, a clamp and/or a thread) and/or a holding element which ensures a distance between the markers and the object (for example in order to assist the visibility of the markers to a marker detection device) and/or marker holders which are mechanically connected to the holding element and which the markers can be attached to.

The disclosure is also directed to a navigation system for computer-assisted surgery. This navigation system preferably comprises the aforementioned computer for processing the data provided in accordance with the computer implemented method as described in any one of the embodiments described herein. The navigation system preferably comprises a detection device for detecting the position of detection points which represent the main points and auxiliary points, in order to generate detection signals and to supply the generated detection signals to the computer, such that the computer can determine the absolute main point data and absolute auxiliary point data on the basis of the detection signals received. A detection point is for example a point on the surface of the anatomical structure which is detected, for example by a pointer. In this way, the absolute point data can be provided to the computer. The navigation system also preferably comprises a user interface for receiving the calculation results from the computer (for example, the position of the main plane, the position of the auxiliary plane and/or the position of the standard plane). The user interface provides the received data to the user as information. Examples of a user interface include a display device such as a monitor, or a loudspeaker. The user interface can use any kind of indication signal (for example a visual signal, an audio signal and/or a vibration signal). One example of a display device is an augmented reality device (also referred to as augmented reality glasses) which can be used as so-called "goggles" for navigating. A specific example of such augmented reality glasses is Google Glass (a trademark of Google, Inc.). An augmented reality device can be used both to input information into the computer of the navigation system by user interaction and to display information outputted by the computer.

The disclosure also relates to a navigation system for computer-assisted surgery, comprising:
a computer for processing the absolute point data and the relative point data;
a detection device for detecting the position of the main and auxiliary points in order to generate the absolute point data and to supply the absolute point data to the computer;
a data interface for receiving the relative point data and for supplying the relative point data to the computer; and
a user interface for receiving data from the computer in order to provide information to the user, wherein the received data are generated by the computer on the basis of the results of the processing performed by the computer.

A navigation system, such as a surgical navigation system, is understood to mean a system which can comprise: at least one marker device; a transmitter which emits electromagnetic waves and/or radiation and/or ultrasound waves; a receiver which receives electromagnetic waves and/or radiation and/or ultrasound waves; and an electronic data processing device which is connected to the receiver and/or the transmitter, wherein the data processing device (for example, a computer) for example comprises a processor (CPU) and a working memory and advantageously an indicating device for issuing an indication signal (for example, a visual indicating device such as a monitor and/or an audio indicating device such as a loudspeaker and/or a tactile indicating device such as a vibrator) and a permanent data memory, wherein the data processing device processes navigation data forwarded to it by the receiver and can advantageously output guidance information to a user via the indicating device. The navigation data can be stored in the permanent data memory and for example compared with data stored in said memory beforehand.

A landmark is a defined element of an anatomical body part which is always identical or recurs with a high degree of similarity in the same anatomical body part of multiple patients. Typical landmarks are for example the epicondyles of a femoral bone or the tips of the transverse processes and/or dorsal process of a vertebra. The points (main points or auxiliary points) can represent such landmarks. A landmark which lies on (for example on the surface of) a characteristic anatomical structure of the body part can also represent said structure. The landmark can represent the anatomical structure as a whole or only a point or part of it. A landmark can also for example lie on the anatomical structure, which is for example a prominent structure. An example of such an anatomical structure is the posterior aspect of the iliac crest. Another example of a landmark is one defined by the rim of the acetabulum, for instance by the centre of said rim. In another example, a landmark represents the bottom or deepest point of an acetabulum, which is derived from a multitude of detection points. Thus, one landmark can for example represent a multitude of detection points. As mentioned above, a landmark can represent an anatomical characteristic which is defined on the basis of a characteristic structure of the body part. Additionally, a landmark can also represent an anatomical characteristic defined by a relative movement of two body parts, such as the rotational centre of the femur when moved relative to the acetabulum.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1A: shows a first embodiment of a single segment of an ultrasound probe according to the first aspect, having a planar transducer surface;
- Fig. 1B: shows a second embodiment of a single segment of an ultrasound probe according to the first aspect, having a one-dimensionally curved transducer surface;
- Fig. 2: shows a first embodiment of an ultrasound probe assembly according to the first aspect, including multiple segments of Fig. 1A;
- Fig. 3: shows a second embodiment of an ultrasound probe assembly according to the first aspect, including multiple segments of Fig. 1B;
- Fig. 4: shows a third embodiment of an ultrasound probe according to the first aspect;
- Fig. 5: shows a fourth embodiment of an ultrasound probe according to the first aspect;
- Fig. 6A and 6B: schematically show how multiple probe segments are assembled to create an ultrasound probe assembly according to the first aspect;
- Fig. 7 to 9: show different buildups for an ultrasound probe according to the first aspect, adapted for specific requirements of different applications.

### DESCRIPTION OF EMBODIMENTS

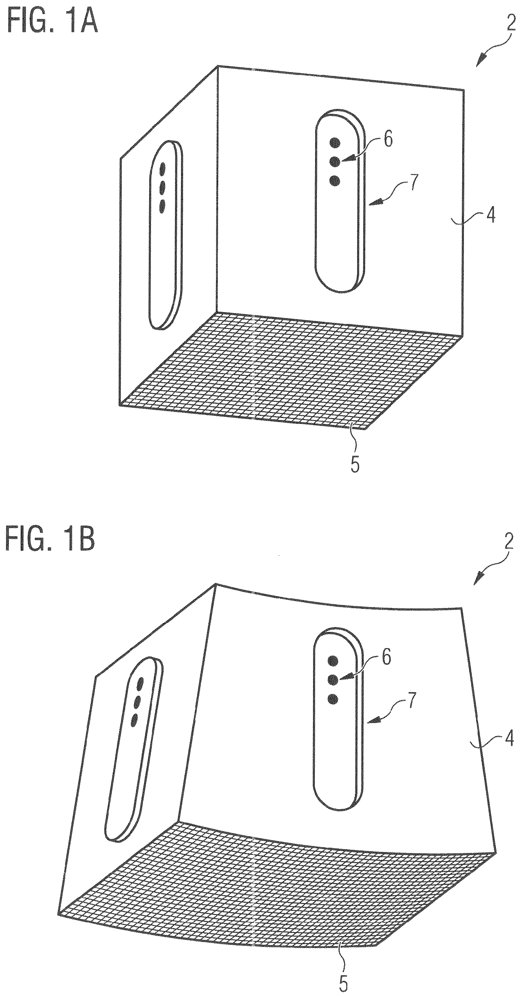

Figure 1A schematically illustrates a first example of an ultrasound probe segment 2 in accordance with the present invention. Segment 2 features a cube-shape, wherein a bottom surface includes a two-dimensional, planar array of transducer elements 5 (e.g. 64 x 64 transducer elements 5). The side-surfaces of the housing 4 each include a mechanical interface 7, i.e. correspondingly formed protrusions or recesses, each of which includes a signal interface 6 being represented by a plurality of electrical contacts.

Figure 1B shows a second exemplary embodiment of the probe segment 2, wherein the bottom surface defined by the two-dimensional array of transducer elements 5 is one-dimensionally curved.

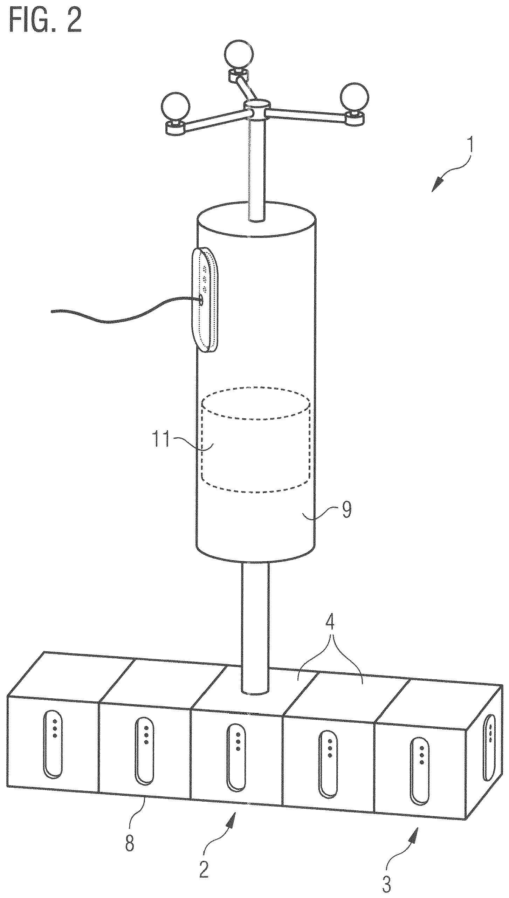

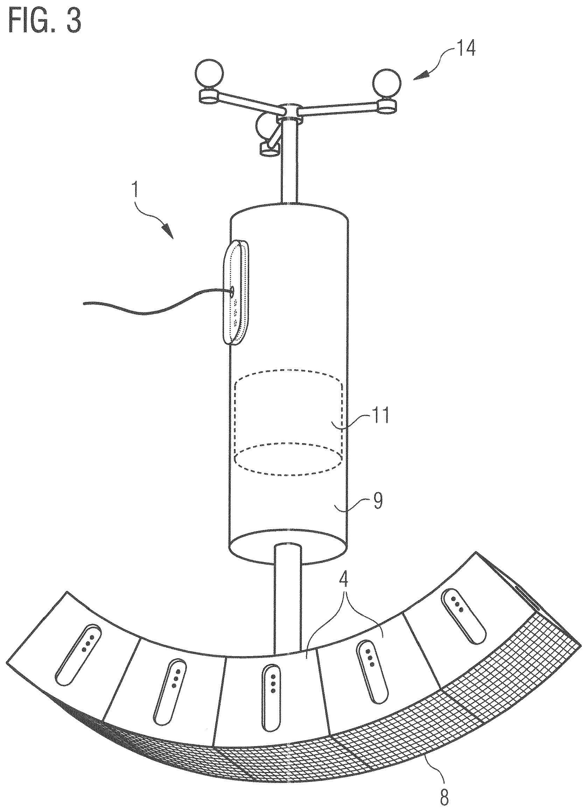

Figures 2 and 3 show an ultrasound probe assembly 1 including five probe segments 2 of Figure 1A and Figure 1B, respectively, which have been put together in a linear fashion so as to create a longitudinal probe-surface 8. A central probe segment 2 features a handle portion 9 that in turn comprises an internal signal unit 11, a signal interface (not indicated) for providing a data connection to an external unit or device, and a tracking marker array 14. The tracking marker array allows for determining and tracking the spatial position of the ultrasound probe assembly 1 such that the ultrasound image generated can be easily registered with the patient's anatomy and/or with image data acquired by other imaging modalities such as CT or MRI. The remaining, auxiliary probe segments 3 connect to the main segment 2 via the mechanical interfaces 7 and the signal interfaces 6 thereof and can therefore also be controlled via the signal unit 11.

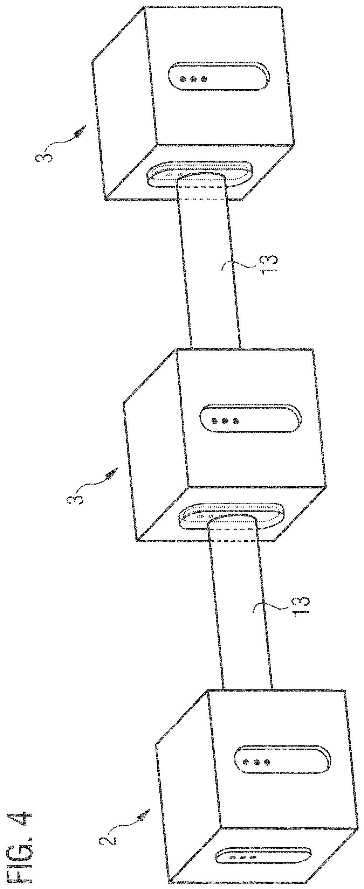

Figure 4 shows an ultrasound probe assembly comprising three individual probe segments 2/3 which are respectively spaced apart from each other via intermediate segments 13. The segments 13 connect between the respective probe segments 2 by engaging the corresponding mechanical interfaces 7 and signal interfaces 6 thereof so as to rigidly but still releasably connect the individual probe segments 2/3 and also in order to provide a data connection therebetween.

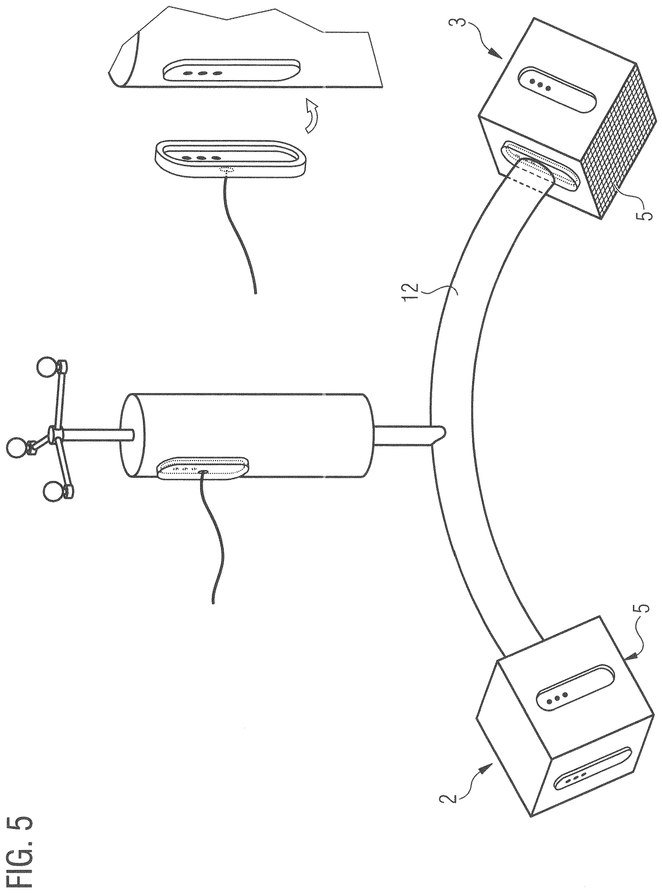

Figure 5 shows an ultrasound probe assembly having a separate handle portion 12 which, just like the intermediate section shown in Figure 4, connects between two separate probe segments 2/3 and holds these segments 2/3 at a specific angle with respect to each other. It becomes apparent from Figure 5 that the handle portion 9 may feature a mechanical interface and a signal interface similar to the individual probe segments 2/3.

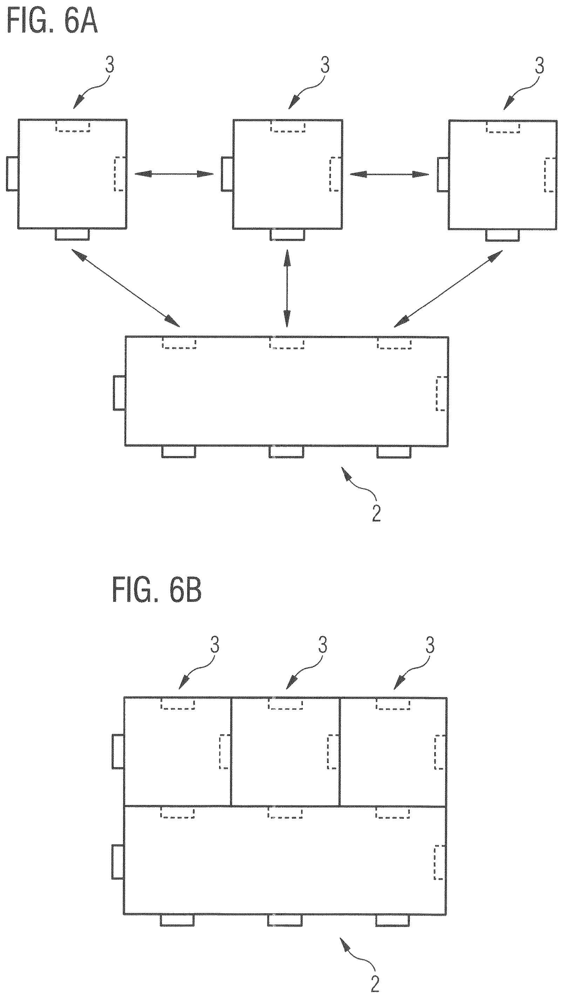

Figure 6A provides a top-view on the individual probe segments 2/3 and schematically illustrates the assembly of multiple probe segments 2/3 to form an ultrasound probe assembly as shown in Figure 6B.

It becomes apparent from Figures 6A and 6B that the segments 2/3 can be assembled in any desired arrangement as the mechanical interfaces thereof which rigidly hold the segments 2/3 together are identical for every probe segment 2/3.

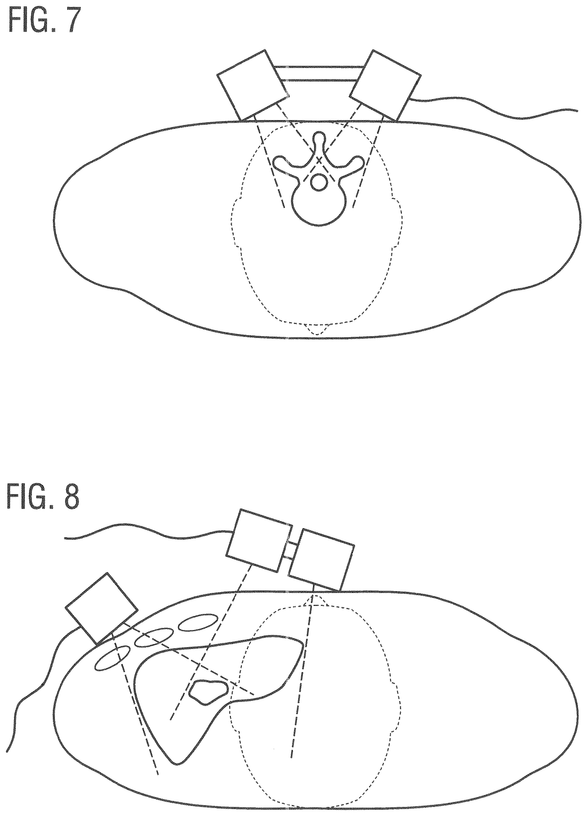

Figures 7 to 9 illustrate different applications for the inventive ultrasound probe assembly. While the segments shown in Figure 7 are rigidly connected to each other via an intermediate element, the ultrasound probe assembly shown in Figure 8 comprises three probe segments, two of which rigidly connect to each other with the third segment being provided separately therefrom. Still, all of the segments receive and sent signals from and to the same signal unit for generating an ultrasound image.

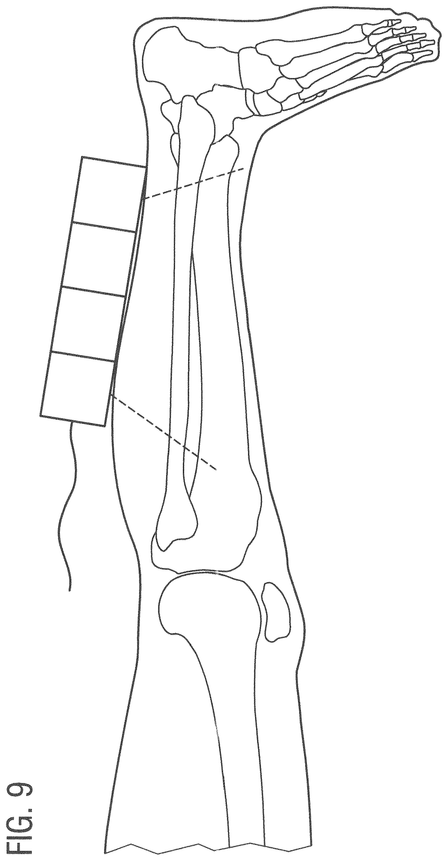

Figure 9 shows a further specific application with four segments being arranged in a linear fashion so as to observe a large volume within a patient's lower leg.

## Claims

1. Ultrasound probe assembly for emitting and/or receiving ultrasound waves, comprising a plurality of selectively and mechanically interconnectable probe segments (2, 3), wherein each one of the probe segments (2, 3) includes:
- a housing (4);
- at least one transducer element (5) which is at least partially received in the housing (4) and is adapted to convert electrical energy in sound energy and/or to convert sound energy in electrical energy;
- at least one signal interface (6) connected to the at least one transducer element (5) and adapted to transmit signals corresponding to the emitted and/or received ultrasound waves;
- at least one mounting interface (7) on the housing (4), which is adapted to transmit a mechanical load to and from the housing (4), and in particular is adapted to connect to a corresponding mounting interface (7) of a similar probe segment (2, 3); **characterized in that** the signal interfaces (6) of at least two probe segments (2, 3) are adapted to interconnect with each other to transmit said signals between the at least two probe segments (2, 3) corresponding to the ultrasound waves emitted and/or received.

2. Ultrasound probe assembly according to claim 1, wherein each one of the probe segments (2, 3) comprises a plurality of transducer elements (5) disposed in a linear or in a two-dimensional arrangement, which in particular define one of:
- a planar probe-surface (8);
- a one-dimensionally curved probe-surface (8);
- a two-dimensionally curved probe-surface (8).

3. Ultrasound probe assembly according to claim 2, wherein at least one of the plurality of transducer elements (5) is adapted to or controlled to convert electrical energy in sound energy, and at least one of the plurality of transducer elements (5) is adapted to or controlled to convert sound energy in electrical energy.

4. Ultrasound probe assembly according to any one of claims 1 to 3, wherein the housing (4) of at least one of the probe segments (2, 3) comprises a handle portion (9) for grasping the ultrasound probe (1) assembly.

5. Ultrasound probe assembly according to any one of claims 1 to 4, wherein said signals are analogue and/or digital signals and the at least one signal interface (6) is adapted to:
- transmit said signals to the probe segment (2, 3), which excite at least one predefined transducer element (5) to emit ultrasound waves; and/or
- transmit said signals from the probe segment (2, 3), which describe ultrasound waves received by at least one predefined transducer element (5).

6. Ultrasound probe assembly according to any one of claims 1 to 5, wherein the probe segments (2, 3) are selectively interconnectable via at least one mounting interface (7), specifically wherein at least two probe segments (2, 3) each include at least one mounting interface (7) which interconnect with each other in such a way that the probe-surface (8) thereby created has a smooth shape across the interconnected segments (2, 3).

7. Ultrasound probe assembly according to claim 6, further comprising a signal unit (11) adapted for generating, processing and/or analyzing said signals, wherein the signal unit (11) is connected to the signal interface (6) of at least one of the plurality of probe segments (2, 3), and adapted to selectively control predefined transducer elements (5) of at least one of the plurality of probe segments (2, 3) to convert electrical energy in sound energy and/or to convert sound energy in electrical energy.

8. Ultrasound probe assembly according to any one of claims 6 and 7, further comprising intermediate segment (13) adapted to releasably connect between at least two of the plurality of probe segments (2, 3), particularly via at least one of the respective signal interfaces (6) and the respective mounting interfaces (7).

9. Ultrasound probe assembly according to any one of claims 6 to 8, further comprising a separate handle segment (12) adapted to releasably connect to at least one of the plurality of probe segments (2, 3), particularly via at the respective mounting interface (7).

10. Ultrasound probe assembly according to any one of claims 6 to 9, wherein
- one first of the plurality of probe segments (2, 3) serves as a main segment (2) which in particular comprises at least one of the handle portion (9), the signal unit (11) and the signal interface (6), and
- at least one second of the plurality of probe segments (2, 3) serves as an auxiliary segment (3) which connects to the main segment and is in particular controlled via the main segment (2).

11. Ultrasound probe assembly according to any one of claims 6 to 10, wherein at least one of the plurality of probe segments (2, 3), particularly at least one of the auxiliary segments (3), includes at least one identification feature which enables the signal unit (11) to determine, particularly via the at least one signal interface (6), at least one property of emission characteristics and/or reception characteristics provided by the respective probe segment (2, 3) connected to the ultrasound probe assembly (1), particularly with respect to a point of reference (14) assigned to the ultrasound probe assembly (1) or a tracking marker (14) of the ultrasound probe assembly (1).

12. Computer implemented method of determining emission characteristics and/or reception characteristics of an ultrasound probe assembly (1) according to any one of claims 1 to 11, the method comprising the following steps:
- transducer emission data is acquired (S1) which describes at least one transducer element (5) adapted to or controlled to convert electrical energy in sound energy, particularly at least one of its characteristics in converting electrical energy in sound energy and its spatial position, specifically its spatial position relative to other transducer elements (5) of the ultrasound probe assembly (1);
- transducer reception data is acquired (S2) which describes at least one transducer element (5) adapted to or controlled to convert sound energy in electrical energy, particularly at least one of its characteristics in converting sound energy in electrical energy and its spatial position, specifically its spatial position relative to other transducer elements (5) of the ultrasound probe assembly (1);
- emission signal data is acquired (S3) which describes at least one signal transmitted to the at least one transducer element (5) adapted to or controlled to convert electrical energy in sound energy;
- reception signal data is acquired (S4) which describes at least one signal received from the at least one transducer element (5) adapted to or controlled to convert sound energy in electrical energy;
- correlation data is determined (S5) based on the transducer emission data, the transducer reception data, the emission reception data and the emission signal data, describing a correlation between the emission characteristics and the reception characteristics provided by the ultrasound probe assembly (1).

13. Computer implemented method according to claim 12 for supporting assembly of an ultrasound probe assembly (1), further comprising the following steps:
- properties data is acquired (S6) which describes at least one desired property of emission characteristics and/or reception characteristics provided by the ultrasound probe assembly (1) according to any one of claims 6 to 11;
- assembly data is determined (S7) based on the properties data and the correlation data, the assembly data describing steps for assembling an ultrasound probe assembly (1) from the least one probe segment (2, 3), which provides the desired property of emission characteristics and/or reception characteristics.

14. Computer program comprising instructions which, when the program is executed by a computer (15), cause the computer (15) to carry out the method according to any one of claims 12 or 13;
and/or a computer-readable storage medium on which the program is stored;
and/or a computer (15) comprising at least one processor and/or the program storage medium, wherein the program is executed by the processor;
and/or a data carrier signal carrying the program;
and/or a data stream comprising the program.

## Patentansprüche

1. Ultraschallsondenanordnung zum Senden und/oder Empfangen von Ultraschallwellen, umfassend eine Vielzahl von wahlweise und mechanisch miteinander verbindbaren Sondensegmenten (2, 3), wobei jedes der Sondensegmente (2, 3) umfasst:
- ein Gehäuse (4);
- mindestens ein Wandlerelement (5), das zumindest teilweise in dem Gehäuse (4) aufgenommen ist und dazu ausgelegt ist, elektrische Energie in Schallenergie umzuwandeln und/oder Schallenergie in elektrische Energie umzuwandeln;
- mindestens eine Signalschnittstelle (6), die mit dem mindestens einen Wandlerelement (5) verbunden ist und dazu ausgelegt ist, Signale zu übertragen, die den ausgesendeten und/oder empfangenen Ultraschallwellen entsprechen;
- mindestens eine Befestigungsschnittstelle (7) am Gehäuse (4), die dazu ausgelegt ist, eine mechanische Belastung zum und vom Gehäuse (4) zu übertragen, und insbesondere dazu ausgelegt ist, mit einer entsprechenden Befestigungsschnittstelle (7) eines gleichartigen Sondensegments (2, 3) verbunden zu werden; **dadurch gekennzeichnet, dass**
die Signalschnittstellen (6) von mindestens zwei Sondensegmenten (2, 3) dazu ausgelegt sind, miteinander verbunden zu werden, um die Signale zwischen den mindestens zwei Sondensegmenten (2, 3) entsprechend den ausgesendeten und/oder empfangenen Ultraschallwellen zu übertragen.

2. Ultraschallsonnenanordnung nach Anspruch 1, wobei jedes der Sondensegmente (2, 3) eine Vielzahl von Wandlerelementen (5) umfasst, die in einer linearen oder zweidimensionalen Anordnung angeordnet sind und insbesondere eines der folgenden Elemente definieren:
- eine ebene Sondenoberfläche (8);
- eine eindimensional gekrümmte Sondenoberfläche (8);
- eine zweidimensional gekrümmte Sondenoberfläche (8).

3. Ultraschallsonde gemäß Anspruch 2, wobei mindestens eines der mehreren Wandlerelemente (5) so ausgelegt oder gesteuert ist, dass es elektrische Energie in Ultraschallenergie umwandelt, und mindestens eines der mehreren Wandlerelemente (5) so ausgelegt oder gesteuert ist, dass es Schallenergie in elektrische Energie umwandelt.

4. Ultraschallsonde gemäß einem der Ansprüche 1 bis 3, wobei das Gehäuse (4) mindestens eines der Sondensegmente (2, 3) einen Griffabschnitt (9) zum Greifen der Ultraschallsonde (1) umfasst.

5. Ultraschallsonde gemäß einem der Ansprüche 1 bis 4, wobei die Signale analoge und/oder digitale Signale sind und die mindestens eine Signalschnittstelle (6) dazu ausgelegt ist:
- die Signale an das Sondensegment (2, 3) zu übertragen, die mindestens ein vordefiniertes Wandlerelement (5) zur Aussendung von Ultraschallwellen anregen; und/oder
- die Signale vom Sondensegment (2, 3) zu übertragen, die von mindestens einem vordefinierten Wandlerelement (5) empfangene Ultraschallwellen beschreiben.

6. Ultraschallsondenanordnung nach einem der Ansprüche 1 bis 5, wobei die Sondensegmente (2, 3) über mindestens eine Befestigungsschnittstelle (7) wahlweise miteinander verbindbar sind, wobei insbesondere mindestens zwei Sondensegmente (2, 3) jeweils mindestens eine Befestigungsschnittstelle (7) aufweisen, die so miteinander verbunden sind, dass die dadurch geschaffene Sondenoberfläche (8) über die miteinander verbundenen Segmente (2, 3) hinweg eine glatte Form aufweist.

7. Ultraschallsondenanordnung gemäß Anspruch 6, die ferner eine Signaleinheit (11) umfasst, die zum Erzeugen, Verarbeiten und/oder Analysieren der Signale ausgelegt ist, wobei die Signaleinheit (11) mit der Signalschnittstelle (6) mindestens eines der mehreren Sondensegmente (2, 3) verbunden ist und zum wahlweisen Steuern vordefinierter Wandlerelemente (5) mindestens eines der mehreren Sondensegmente (2, 3) ausgelegt ist, um elektrische Energie in Schallenergie umzuwandeln und/oder Schallenergie in elektrische Energie umzuwandeln.

8. Ultraschallsondenanordnung nach einem der Ansprüche 6 und 7, die ferner ein Zwischensegment (13) umfasst, das dazu ausgestaltet ist, zwischen mindestens zwei der mehreren Sondensegmente (2, 3) mit diesen lösbar verbunden zu werden, insbesondere über mindestens eine der jeweiligen Signalschnittstellen (6) und die jeweiligen Befestigungsschnittstellen (7).

9. Ultraschallsondenanordnung nach einem der Ansprüche 6 bis 8, die ferner ein separates Griffsegment (12) umfasst, das dazu ausgelegt ist, lösbar mit mindestens einem der mehreren Sondensegmente (2, 3) verbunden zu werden, insbesondere über die jeweilige Befestigungsschnittstelle (7).

10. Ultraschallsondenanordnung nach einem der Ansprüche 6 bis 9, wobei
- ein erstes der mehreren Sondensegmente (2, 3) als Hauptsegment (2) dient, das insbesondere mindestens eines der folgenden Elemente umfasst: den Griffabschnitt (9), die Signaleinheit (11) und die Signalschnittstelle (6), und
- mindestens ein zweites der mehreren Sondensegmente (2, 3) als Hilfssegment (3) dient, das mit dem Hauptsegment verbunden ist und insbesondere über das Hauptsegment (2) gesteuert wird.

11. Ultraschallsondenanordnung nach einem der Ansprüche 6 bis 10, wobei mindestens eines der mehreren Sondensegmente (2, 3), insbesondere mindestens eines der Hilfssegmente (3), mindestens ein Identifikationsmerkmal aufweist, das es der Signaleinheit (11) ermöglicht, insbesondere über die mindestens eine Signalschnittstelle (6) mindestens eine Eigenschaft von Emissionseigenschaften und/oder Empfangseigenschaften zu bestimmen, die von dem jeweiligen mit der Ultraschallsondenanordnung (1) verbundenen Sondensegment (2, 3) bereitgestellt werden, insbesondere in Bezug auf einen der Ultraschallsondenanordnung (1) zugeordneten Bezugspunkt (14) oder eine Tracking-Markierung (14) der Ultraschallsondenanordnung (1).

12. Computerimplementiertes Verfahren zum Bestimmen von Emissionseigenschaften und/oder Empfangseigenschaften einer Ultraschallsondenanordnung (1) gemäß einem der Ansprüche 1 bis 11, wobei das Verfahren die folgenden Schritte umfasst:
- Erfassen (S1) von Wandler-Emissionsdaten, die mindestens ein Wandlerelement (5) beschreiben, das dazu ausgelegt oder gesteuert ist, elektrische Energie in Schallenergie umzuwandeln, insbesondere mindestens eine seiner Eigenschaften bei der Umwandlung von elektrischer Energie in Schallenergie und seine räumliche Position, insbesondere seine räumliche Position relativ zu anderen Wandlerelementen (5) der Ultraschallsondenanordnung (1);
- Erfassen von Wandlerempfangsdaten (S2), die mindestens ein Wandlerelement (5) beschreiben, das dazu ausgelegt oder so gesteuert ist, Schallenergie in elektrische Energie umzuwandeln, insbesondere mindestens eine seiner Eigenschaften bei der Umwandlung von Schallenergie in elektrische Energie und seine räumliche Position, insbesondere seine räumliche Position relativ zu anderen Wandlerelementen (5) der Ultraschallsondenanordnung (1);
- Erfassen von Emissionssignaldaten (S3), die mindestens ein Signal beschreiben, das an das mindestens eine Wandlerelement (5) übertragen wird, das dazu ausgelegt oder gesteuert ist, elektrische Energie in Schallenergie umzuwandeln;
- Erfassen von Empfangssignaldaten (S4), die mindestens ein Signal beschreiben, das von dem mindestens einen Wandlerelement (5) empfangen wird, das dafür ausgelegt oder so gesteuert ist, Schallenergie in elektrische Energie umzuwandeln;
- Bestimmen von Korrelationsdaten (S5) auf der Grundlage der Wandler-Emissionsdaten, der Wandler-Empfangsdaten, der Emissionssignaldaten und der Empfangssignaldaten, die eine Korrelation zwischen den Sendeeigenschaften und den Empfangseigenschaften der Ultraschallsonde (1) beschreiben.

13. Computerimplementiertes Verfahren gemäß Anspruch 12 zur Unterstützung der Montage einer Ultraschallsonde (1), das ferner die folgenden Schritte umfasst:
- Erfassen von Eigenschaftsdaten (S6), die mindestens eine gewünschte Eigenschaft der von der Ultraschallsondenanordnung (1) bereitgestellten Emissionscharakteristika und/oder Empfangscharakteristika gemäß einem der Ansprüche 6 bis 11 beschreiben;
- Bestimmen von Montagedaten basierend auf den Eigenschaftsdaten und den Korrelationsdaten (S7), wobei die Montagedaten Schritte zum Montieren einer Ultraschallsondenanordnung (1) aus dem mindestens einen Sondensegment (2, 3) beschreiben, das die gewünschte Eigenschaft der Emissionseigenschaften und/oder Empfangseigenschaften bereitstellt.

14. Computerprogramm, das Befehle umfasst, die, wenn das Programm von einem Computer (15) ausgeführt wird, den Computer (15) veranlassen, das Verfahren gemäß einem der Ansprüche 12 oder 13 auszuführen;
und/oder ein computerlesbares Speichermedium, auf dem das Programm gespeichert ist;
und/oder einen Computer (15) mit mindestens einem Prozessor und/oder dem Programmspeichermedium, wobei das Programm vom Prozessor ausgeführt wird;
und/oder ein Datenträgersignal, das das Programm trägt;
und/oder einen Datenstrom, der das Programm umfasst.

## Revendications

1. Ensemble de sonde échographique destiné à émettre et/ou recevoir des ondes ultrasonores, comprenant une pluralité de segments de sonde (2, 3) pouvant être interconnectés de manière sélective et mécanique, chacun des segments de sonde (2, 3) comprenant :
- un boîtier (4),
- au moins un élément transducteur (5) qui est au moins partiellement logé dans le boîtier (4) et qui est adapté à convertir l'énergie électrique en énergie sonore et/ou à convertir l'énergie sonore en énergie électrique,
- au moins une interface de signaux (6) raccordée à l'au moins un élément transducteur (5) et adaptée à transmettre des signaux correspondant aux ondes ultrasonores émises et/ou reçues,
- au moins une interface de montage (7) sur le boîtier (4), qui est adaptée à transmettre une charge mécanique dirigée vers le boîtier (4) ou provenant de celui-ci, et qui est notamment adaptée à être raccordée à une interface de montage (7) correspondante d'un segment de sonde (2, 3) similaire ;
**caractérisé en ce que** les interfaces de signaux (6) d'au moins deux segments de sonde (2, 3) sont adaptées à s'interconnecter entre elles afin de transmettre lesdits signaux entre les au moins deux segments de sonde (2, 3) correspondant aux ondes ultrasonores émises et/ou reçues.

2. Ensemble de sonde échographique selon la revendication 1, dans lequel chacun des segments de sonde (2, 3) comprend une pluralité d'éléments transducteurs (5) disposés selon un agencement linéaire ou bidimensionnel, qui définissent notamment :
- une surface de sonde (8) plane,
- une surface de sonde (8) à courbure unidimensionnelle, et/ou
- une surface de sonde (8) à courbure bidimensionnelle.

3. Ensemble de sonde échographique selon la revendication 2, dans lequel au moins un élément de la pluralité d'éléments transducteurs (5) est adapté à convertir l'énergie électrique en énergie sonore ou est commandé pour le faire, et au moins un élément de la pluralité d'éléments transducteurs (5) est adapté à convertir l'énergie sonore en énergie électrique ou est commandé pour le faire.

4. Ensemble de sonde échographique selon l'une quelconque des revendications 1 à 3, dans lequel le boîtier (4) d'au moins un des segments de sonde (2, 3) comprend une portion formant poignée (9) pour saisir l'ensemble de sonde échographique (1).

5. Ensemble de sonde échographique selon l'une quelconque des revendications 1 à 4, dans lequel lesdits signaux sont des signaux analogiques et/ou numériques et l'au moins une interface de signaux (6) est adaptée à :
- transmettre lesdits signaux au segment de sonde (2, 3), lesquels excitent au moins un élément transducteur (5) prédéfini afin qu'il émette des ondes ultrasonores, et/ou
- transmettre lesdits signaux provenant du segment de sonde (2, 3), lesquelles décrivent des ondes ultrasonores reçues par au moins un élément transducteur (5) prédéfini.

6. Ensemble de sonde échographique selon l'une quelconque des revendications 1 à 5, dans lequel les segments de sonde (2, 3) peuvent être interconnectés de manière sélective par l'intermédiaire d'au moins une interface de montage (7) ; au moins deux segments de sonde (2, 3) comprenant plus particulièrement chacun au moins une interface de montage (7), lesquelles interfaces de montage s'interconnectent entre elles de façon que la surface de sonde (8) ainsi créée présente une forme lisse sur les segments (2, 3) interconnectés.

7. Ensemble de sonde échographique selon la revendication 6, comprenant en outre une unité de signaux (11) adaptée à générer, traiter et/ou analyser lesdits signaux, l'unité de signaux (11) étant raccordée à l'interface de signaux (6) d'au moins un segment de la pluralité de segments de sonde (2, 3) et adaptée à commander de manière sélective des éléments transducteurs (5) prédéfinis d'au moins un segment de la pluralité de segments de sonde (2, 3) afin qu'il convertisse l'énergie électrique en énergie sonore et/ou l'énergie sonore en énergie électrique.

8. Ensemble de sonde échographique selon l'une quelconque des revendications 6 et 7, comprenant en outre un segment intermédiaire (13) adapté à être raccordé de manière amovible entre au moins deux segments de la pluralité de segments de sonde (2, 3), notamment par l'intermédiaire d'au moins une des interfaces de signaux (6) respectives et des interfaces de montage (7) respectives.

9. Ensemble de sonde échographique selon l'une quelconque des revendications 6 à 8, comprenant en outre un segment de poignée (12) séparé adapté à être raccordé de manière amovible à au moins un segment de la pluralité de segments de sonde (2, 3), notamment par l'intermédiaire de l'interface de montage (7) respective.

10. Ensemble de sonde échographique selon l'une quelconque des revendications 6 à 9, dans lequel
- un premier segment de la pluralité de segments de sonde (2, 3) sert de segment principal (2), lequel comprend notamment au moins une portion formant poignée (9), l'unité de signaux (11) et l'interface de signaux (6), et
- au moins un deuxième segment de la pluralité de segments de sonde (2, 3) sert de segment auxiliaire (3), lequel est raccordé au segment principal et est notamment commandé par l'intermédiaire du segment principal (2).

11. Ensemble de sonde échographique selon l'une quelconque des revendications 6 à 10, dans lequel au moins un segment de la pluralité de segments de sonde (2, 3), notamment au moins un des segments auxiliaires (3), comprend au moins un élément d'identification qui permet à l'unité de signaux (11) de déterminer, notamment par l'intermédiaire de l'au moins une interface de signaux (6), au moins une propriété des caractéristiques d'émission et/ou des caractéristiques de réception fournies par le segment de sonde (2, 3) respectif raccordé à l'ensemble de sonde échographique (1), notamment par rapport à un point de référence (14) attribué à l'ensemble de sonde échographique (1) ou à un marqueur de suivi (14) de l'ensemble de sonde échographique (1).

12. Procédé mis en œuvre par ordinateur pour déterminer les caractéristiques d'émission et/ou les caractéristiques de réception d'un ensemble de sonde échographique (1) selon l'une quelconque des revendications 1 à 11, le procédé comprenant les étapes suivantes :
- l'acquisition (S1) de données d'émission de transducteur qui décrivent au moins un élément transducteur (5) adapté à convertir l'énergie électrique en énergie sonore ou commandé pour le faire, notamment au moins une de ses caractéristiques de conversion d'énergie électrique en énergie sonore et sa position spatiale, plus particulièrement sa position spatiale par rapport à d'autres éléments transducteurs (5) de l'ensemble de sonde échographique (1),
- l'acquisition (S2) de données de réception de transducteur qui décrivent au moins un élément transducteur (5) adapté à convertir l'énergie sonore en énergie sonore électrique ou commandé pour le faire, notamment au moins une de ses caractéristiques de conversion d'énergie sonore en énergie électrique et sa position spatiale, plus particulièrement sa position spatiale par rapport à d'autres éléments transducteurs (5) de l'ensemble de sonde échographique (1),
- l'acquisition (S3) de données de signal d'émission qui décrivent au moins un signal transmis à l'au moins un élément transducteur (5) adapté à convertir l'énergie électrique en énergie sonore ou commandé pour le faire,
- l'acquisition (S4) de données de signal de réception qui décrivent au moins un signal reçu en provenance de l'au moins un élément transducteur (5) adapté à convertir l'énergie sonore en énergie électrique ou commandé pour le faire,
- la détermination (S5) de données de corrélation en fonction des données d'émission de transducteur, des données de réception de transducteur, des données d'émission-réception et des données de signal d'émission, décrivant une corrélation entre les caractéristiques d'émission et les caractéristiques de réception fournies par l'ensemble de sonde échographique (1).

13. Procédé mis en œuvre par ordinateur selon la revendication 12 pour faciliter l'assemblage d'un ensemble de sonde échographique (1), comprenant en outre les étapes suivantes :
- l'acquisition (S6) de données de propriété qui décrivent au moins une propriété souhaitée des caractéristiques d'émission et/ou des caractéristiques de réception fournies par l'ensemble de sonde échographique (1) selon l'une quelconque des revendications 6 à 11,
- la détermination (S7) de données d'assemblage en fonction des données de propriété et des données de corrélation, les données d'assemblage décrivant des étapes d'assemblage d'un ensemble de sonde échographique (1) à partir d'au moins un segment de sonde (2, 3), qui fournit la propriété souhaitée des caractéristiques d'émission et/ou des caractéristiques de réception.

14. Programme informatique comprenant des instructions qui, lorsque le programme est exécuté par un ordinateur (15), amènent l'ordinateur (15) à mettre en œuvre le procédé selon l'une quelconque des revendications 12 et 13,
et/ou support de stockage lisible par ordinateur sur lequel est stocké le programme,
et/ou ordinateur (15) comprenant au moins un processeur et/ou le support de stockage de programme, ledit programme étant exécuté par le processeur,
et/ ou signal porteur de données portant le programme,
et/ou flux de données comprenant le programme.
